# DEMANDE DE BREVET EUROPEEN

(11) **EP 2 116 220 A1**
(43) Date de publication de la demande: **11.11.2009**
(21) Numéro de dépôt: 09158076.1
(22) Date de dépôt: 16.04.2009
(51) Int. Cl.: A61K 8/06, A61K 8/35, A61K 8/41, A61K 8/92, A61Q 5/00, A61Q 5/02, A61Q 5/12

(54) **Composition cosmétique comprenant une émulsion obtenue par un procédé PIT**

(30) Priorité: 28.04.2008 FR 0852848
(71) Demandeur: L'Oreal, 75008 Paris (FR)
(72) Inventeur: Mathonneau, Estelle, 75010 Paris (FR)
(74) Mandataire: Dossmann, Gérard

(57) **Abrégé**

L'invention porte sur une composition cosmétique aqueuse pour le traitement des matières kératiniques, telles que les cheveux, comprenant une émulsion directe (Al) obtenue par un procédé PIT et un ou plusieurs polymères cationiques, caractérisée par le fait que l'émulsion directe (Al) comporte :
a) une phase lipophile comprenant un ou plusieurs alcools gras,
b) un système émulsionnant comprenant un ou plusieurs tensioactifs non-ioniques oxyéthylénés et/ou glycérolés de HLB allant de 8 à 18, et
c) une phase aqueuse,
le rapport pondéral de la quantité d'alcool gras sur la quantité de polymères cationiques étant supérieur à 1,
ainsi que son procédé de préparation, un procédé de traitement des matières kératiniques et son utilisation dans le domaine capillaire.

## Description

La présente invention se rapporte à une composition cosmétique aqueuse comprenant une émulsion directe, obtenue par inversion de phase, et à ses utilisations dans le domaine capillaire.

La fabrication d'émulsions directes concentrées en huile est souvent difficile, et les émulsions obtenues sont souvent instables. Ces émulsions sont classiquement obtenues par voie mécanique, par exemple par émulsification avec un rotor stator ou à l'aide d'un homogénéisateur haute-pression, parce qu'il faut beaucoup d'énergie mécanique pour diviser la phase dispersée en petites gouttes. Pour stabiliser ces émulsions, on y ajoute généralement des tensioactifs émulsionnants du type huile-dans-eau, c'est-à-dire de HLB (HLB = Hydrophilic Lipophilic Balance) allant de 8 à 18, émulsionnants qui, grâce à leur structure amphiphile, se placent à l'interface phase huileuse / phase aqueuse, et stabilisent ainsi les gouttelettes d'huile dispersées. Malgré la présence d'émulsionnants, les émulsions peuvent avoir tendance à se déstabiliser (coalescence puis séparation des phases aqueuse et huileuse avec relargage d'huile). Pour améliorer la stabilité de ces émulsions, on peut augmenter la concentration en émulsionnants. Toutefois, une forte concentration en émulsionnants peut conduire à un toucher rêche, collant ou poisseux, ainsi qu'à des problèmes d'innocuité vis-à-vis de la peau, des yeux et du cuir chevelu.

Pour résoudre les problèmes de stabilité des émulsions directes classiques, il a été proposé de réaliser des émulsions directes obtenues par inversion de phase en température (émulsions PIT), dans lesquelles la taille moyenne des globules constituant la phase huileuse est comprise, de préférence entre 0,1 à 4 µm (100 à 4000 nm). Le principe d'émulsification par inversion de phase en température (en Anglais : Phase Inversion Temperature ou PIT) est, dans son principe, bien connu de l'homme de l'art. Il a été décrit en 1968 par K. Shinoda (J. Chem. Soc. Jpn., 1968, 89, 435). Il a été montré que cette technique d'émulsification permet d'obtenir des émulsions fines stables (K. Shinoda et H. Saito, J. Colloïd Interface Sci., 1969,30, 258). Cette technologie a été appliquée en cosmétique dès 1972 par Mitsui et al. (Application of the phase-inversion- temperature method to the emulsification of cosmetics ; T. Mitsui, Y. Machida and F. Harusawa, American. Cosmet. Perfum., 1972, 87, 33).

Le principe de cette technique est le suivant : on réalise le mélange d'une phase aqueuse et d'une phase huileuse, que l'on porte à une température supérieure à la température PIT, température d'inversion de phase du système, qui est la température à laquelle l'équilibre entre les propriétés hydrophile et lipophile du ou des émulsionnants mis en oeuvre est atteint. A température élevée, c'est-à-dire supérieure à la température d'inversion de phase (>PIT), l'émulsion est de type eau-dans-huile. Au cours de son refroidissement, cette émulsion s'inverse à la température d'inversion de phase, pour devenir une émulsion de type huile-dans-eau, et ceci en étant passée auparavant par un état de microémulsion. Ce procédé permet d'obtenir facilement des émulsions, dont le diamètre moyen en volume des gouttelettes de l'émulsion est inférieur à 4 µm.

L'utilisation de ce type d'émulsion est connue dans le domaine des compositions cosmétiques solaires ou dans le domaine de la coloration capillaire, ces émulsions présentant l'avantage d'être plus stables au stockage.

Or, il subsiste encore un besoin d'améliorer les propriétés cosmétiques et conditionnantes, telle que le toucher des cheveux traités par ces compositions sous forme d'émulsions PIT.

La demanderesse a trouvé de manière surprenante que le choix de composés particuliers pour réaliser ces émulsions permettait d'améliorer l'efficacité des compositions au niveau des propriétés cosmétiques, notamment en terme de toucher.

La présente invention a donc pour objet une composition cosmétique aqueuse pour le traitement des matières kératiniques, telles que les cheveux, comprenant une émulsion directe (A1) obtenue par un procédé PIT et un ou plusieurs polymères cationiques, caractérisée par le fait que l'émulsion directe (A1) comporte :
a) une phase lipophile comprenant un ou plusieurs alcools gras,
b) un système émulsionnant comprenant un ou plusieurs tensio-actifs non-ioniques oxyéthylénés et/ou glycérolés de HLB allant de 8 à 18, et
c) une phase aqueuse,
le rapport pondéral de la quantité d'alcools gras sur la quantité de polymères cationiques étant supérieur à 1.

Il va de soi que la composition de l'invention constitue un milieu cosmétiquement acceptable. Par "milieu cosmétiquement acceptable", on entend un milieu convenant à une application cosmétique pour les cheveux, le cuir chevelu, la peau ou les yeux.

Par émulsion directe, on entend au sens de la présente invention, une émulsion à phase continue aqueuse correspondant à la dispersion d'une phase lipophile dans ladite phase aqueuse continue. On parle souvent de manière simplifiée d'émulsion H/E (huile dans l'eau).

Par composition finale, on entend la composition résultant du mélange de l'émulsion PIT et de la composition aqueuse comprenant le polymère cationique.

Les compositions finales selon l'invention se caractérisent par :
- leur aspect qui peut aller de l'opaque au translucide voire transparent,
- le pH qui va de 3 à 8,
- la petite taille des gouttelettes de phase lipophile,
- lorsqu'elles sont rincées, une bonne ré-émulsification de la phase lipophile lors du rinçage à l'eau pour limiter le résidu lipophile sur le cuir chevelu ou les cheveux.

En outre, ces compositions sont agréables à utiliser du fait de la phase aqueuse externe et elles allient ainsi efficacité et agrément cosmétique. Le toucher est non gras à l'application et la pénétration de la composition est rapide dans le cheveu.

Les compositions peuvent être utilisées de manière différente en fonction des applications souhaitées : elles peuvent être par exemple utilisées en tant que shampooing ou utilisées en tant que soins capillaires, et en particulier d'après-shampooings, en post ou pré-traitement, de façon rincée ou non rincée.

La composition selon l'invention de préférence ne comporte pas de filtre solaire. De préférence, elle ne contient pas de colorants capillaires directs ou d'oxydation apportant une coloration aux fibres kératiniques.

A moins d'une indication différente, les bornes des gammes de valeurs qui sont données dans le cadre de la présente invention sont incluses dans ces gammes.

### Phase lipophile

On entend par phase lipophile selon la présente invention, la phase contenant les composés lipophiles que sont les alcools gras de l'invention, et éventuellement les huiles (constituants lipophiles liquides à température ambiante), les gommes, les pâtes et les cires.

Les émulsionnants, et éventuellement co-émulsionnants du système émulsionnant ne font pas partie de la phase lipophile telle que définie ci-dessus.

Par alcools gras au sens de la présente invention, on entend des composés de formule suivante : R-OH

R désignant une chaîne hydrocarbonée linéaire ou ramifiée, saturée ou insaturée, comportant de 12 à 30 atomes de carbone. Ces alcools gras peuvent être choisis notamment parmi les alcools oléique, laurique, palmitique, myristique, isomyristylique, béhénylique, stéarylique, isostéarylique, cétylique, isocétylique linoléique, linolénique, arachidonique, isoarachidylique, ricinoléique, l'octyldodécanol, ou leur mélange, comme par exemple l'alcool cétylstéarylique.

Parmi lesdits alcools, on préfère plus particulièrement utiliser l'alcool cétylstéarylique.

Le ou les alcools gras décrits ci-dessus sont de préférence présents en une quantité allant de 0.2 à 40 %, de préférence de 2 à 20 % et mieux de 3 à 11 % en poids par rapport au poids total de la composition finale.

La phase lipophile peut également comprendre d'autres corps gras que les alcools gras mentionnés ci-dessus.

Par corps gras, on entend au sens de la présente invention un composé organosiliconé insoluble dans l'eau ou un composé organique non siliconé non polymérique insoluble dans l'eau.

De préférence pour les composés organiques non siliconés, la pression de vapeur du composé à 25°C est inférieure à 1 mm de Hg.

Selon l'invention, les corps gras préférés sont des huiles et des cires tels que, par exemple, des esters gras et des acides gras.

Par huile, on entend une substance liquide à température ambiante, par exemple de 25°C, et sous pression atmosphérique, et insoluble dans l'eau. Par insoluble dans l'eau, on entend au sens de la présente invention un composé dont la solubilité à pH spontanée dans l'eau à 25°C et à pression atmosphérique est inférieur à 1% et de préférence inférieure à 0,5%. Les huiles ont une température de fusion inférieure à 5°C et une viscosité inférieure à 500cps à 25°C et à un taux de cisaillement de 1s⁻¹.

Les huiles sont choisies de préférence parmi les huiles végétales, les huiles animales, les huiles minérales, les huiles de synthèse, les huiles siliconées, les esters d'acides gras liquides, les acides gras liquides et les amides d'acide gras liquides.

Comme huile végétale, on peut notamment mentionner l'huile d'amande douce, l'huile d'avocat, l'huile de ricin, l'huile d'olive, la cire liquide de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, l'huile de palme, l'huile de noyau d'abricot et l'huile de calophyllum.

Comme huile animale, on peut notamment citer le perhydrosqualène.

Comme huile minérale, on peut citer par exemple une huile de paraffine et l'huile de vaseline.

Comme huile de synthèse, on peut citer par exemple le squalane, les poly(α-oléfines) comme l'isododécane ou l'isohexadécane, les huiles végétales transestérifiées et les huiles fluorées.

Comme huiles siliconées, on peut notamment citer les polydiméthylsiloxanes cycliques (nom INCI cyclométhicone) tels que le décaméthyl pentacyclosiloxane et les polydiméthylsiloxanes linéaires de faible viscosité, de préférence inférieure à 1000 cSt à 25°C.

A titre d'exemples d'esters gras huileux, on peut notamment citer les composés de formule RₐCOOR_{b} dans laquelle Rₐ représente le reste d'un acide supérieur linéaire ou ramifié, hydroxylé ou non, saturé ou non, comportant de 4 à 29 atomes de carbone et R_{b} représente une chaîne hydrocarbonée linéaire ou ramifiée, saturée ou non contenant de 3 à 30 atomes de carbone, le nombre total d'atomes de carbone de l'ester étant supérieur à 10. A titre d'exemples, on peut notamment citer l'huile de Purcellin (octanoate de stéaryle), le myristate d'isopropyle, le palmitate d'isopropyle, le stéarate de butyle, le laurate d'hexyle, l'isononanoate d'isononyle, le palmitate de 2-éthylhexyle, le laurate de 2-hexyldécyle, le palmitate de 2-octyldécyle, le myristate de 2-octyldodécyle, le néopentanoate d'isostéaryle ou le néopentanoate de tridécyle.

A titre d'exemples d'acide gras huileux, on peut notamment citer l'acide oléique, l'acide linoléique, l'acide ricinoléique, l'acide isotéarique et l'acide dilinoléique, et de préférence l'acide oléique et l'acide linoléique.

On peut notamment citer les amides d'acide gras non alcoxylés huileux.

Par cire, on entend un composé gras lipophile, solide ou pâteux à température ambiante (20 à 25°C), à changement d'état solide/liquide réversible, ayant une température de fusion supérieure à environ 40°C et pouvant aller jusqu'à 200°C. Elle présente généralement une dureté supérieure à 0,5 MPa pour les solides et une dureté comprise entre 0,001 et 0,5 MPa pour les pâtes, et présentant à la température de fusion une organisation cristalline anisotrope.

La dureté de la cire est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

A titre de cires utilisables dans la présente invention, on peut notamment citer les cires d'origine animale, végétale, minérale ou synthétique telles que la cire d'abeille, le spermaceti, les cires fluorées et perfluorées, les cires de lanoline, de lanoline hydrogénée et de lanoline acétylée ; les cires de Candellila, d'Ouricury, de Carnauba, du Japon, le beurre de cacao, les cires de fibres de liège ou de canne à sucre, la cire de son de riz, la cire de sapin, la cire de coton ; les cires microcristallines, la cire de paraffine, le pétrolatum, la vaseline, l'ozokérite, la cire de montan ; les huiles hydrogénées ayant une température de fusion supérieure à environ 40°C, comme l'huile de jojoba hydrogénée ; les cires de polyéthylène et les cires obtenues par synthèse de Fischer-Tropsch.

Ces cires peuvent être également choisies parmi les alcools gras cireux, les esters gras cireux.

Les esters gras cireux sont des esters d'acide gras, c'est-à-dire des esters d'acides carboxyliques comportant au moins 10 atomes de carbone et d'un monoalcool ou d'un polyol. Les esters gras cireux utilisables dans la composition selon l'invention peuvent être des mono, des di ou des triesters. Les acides carboxyliques comportent de préférence de 10 à 30 atomes de carbone et plus particulièrement de 12 à 24 atomes de carbone. Les monoalcools ou polyols comportent de préférence de 10 à 30 atomes de carbone et plus particulièrement de 12 à 24 atomes de carbone. A titre d'exemples d'esters cireux, on peut citer le myristate de myristyle et le stéarate de stéaryle.

Les acides gras cireux utilisables dans la composition selon l'invention comportent de préférence de 12 à 24 atomes de carbone et peuvent être saturés ou insaturés, ramifiés ou non, et comporter une ou plusieurs fonctions hydroxy. A titre d'exemples d'acide cireux, on peut citer l'acide laurique, l'acide stéarylique, l'acide cétylique et l'acide béhénique.

A titre d'amides cireux, on peut notamment citer les céramides et en particulier la Noléyldihydrosphingosine.

### Système émulsionnant

Le système émulsionnant utilisé dans la composition selon l'invention comprend un ou plusieurs tensioactifs non-ioniques oxyéthylénés et/ou glycérolés de HLB allant de 8 à 18, et de préférence de 10 à 16.

Les émulsionnants présentent la particularité de faciliter la dispersion de deux phases insolubles l'une dans l'autre. La HLB (Hydrophilic Lipophilic balance) est le rapport entre la partie hydrophile et la partie lipophile dans leur molécule. Ce terme HLB est bien connu de l'homme du métier et est décrit dans "The HLB system. A time-saving guide to Emulsifier Selection" (published by ICI Americas Inc ; 1984).

Le système émulsionnant peut comprendre en particulier un ou plusieurs composés choisis parmi les alcools gras éthoxylés, les esters d'acides gras et de PEG, les glycérides partiels d'acides gras éthoxylés, les triglycérides d'acides gras polyglycérolés et leurs dérivés éthoxylés.

Les alcools gras éthoxylés sont de préférence choisis parmi les alcools gras en C18 à C22 et possédant de 6 à 12, et de préférence de 8 à 12 moles d'oxyde d'éthylène.

Comme alcools gras éthoxylés selon l'invention, on peut notamment citer par exemple les produits d'addition d'oxyde d'éthylène avec l'alcool béhénylique, notamment ceux comportant de 6 à 12 groupes oxyéthylénés (par exemple Beheneth-9 ou Beheneth-10 selon la dénomination du CTFA) ; les produits d'addition d'oxyde d'éthylène avec l'alcool stéarylique, notamment ceux comportant de 6 à 12 groupes oxyéthylénés (par exemple Steareth-9 selon la dénomination du CTFA) ; les produits d'addition d'oxyde d'éthylène avec l'alcool isostéarylique, notamment ceux comportant de 6 à 12 groupes oxyéthylénés (Isosteareth-9 selon la dénomination du CTFA) ; et leurs mélanges.

A titre de tensio-actifs non-ioniques de HLB allant de 8 à 18, la composition peut également comprendre des alcools gras oxyéthylénés différents de ceux décrits ci-dessus à savoir, les produits d'addition d'oxyde d'éthylène avec l'alcool laurylique, notamment ceux comportant de 9 à 50 groupes oxyéthylénés (Laureth-9 à Laureth-50 en noms CTFA) ; les produits d'addition d'oxyde d'éthylène avec l'alcool cétéarylique ou cétylstéarylique (mélange d'alcool cétylique et d'alcool stéarylique), notamment ceux comportant de 9 à 30 groupes oxyéthylénés (Ceteareth-9 à Ceteareth-30 en noms CTFA) ; les produits d'addition d'oxyde d'éthylène avec l'alcool cétylique, notamment ceux comportant de 9 à 30 groupes oxyéthylénés (Ceteth-9 à Ceteth-30 en noms CTFA) ; et leurs mélanges.

De préférence, l'alcool gras éthoxylé est le beheneth-10.

Les esters d'acide gras et de PEG sont de préférence choisis parmi les composés de formule (I) suivante :

R-COO-(CH₂-CH₂-O)ₘH (I)

où R est une chaîne hydrocarbonée linéaire ou ramifiée, saturée ou insaturée, ayant de 10 à 24 atomes de carbone, et m est un nombre entier allant de 8 à 50.

Comme esters d'acides gras et de PEG, on peut citer par exemple les produits d'esterification avec les acides laurique, palmitique, stéarique ou béhénique, et leurs mélanges et avec l'oxyde d'éthylène, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA : PEG-9 palmitate à PEG-50 palmitate) ; les stéarates de PEG-9 à PEG-50 (en noms CTFA : PEG-9 stéarate à PEG-50 stearate) ; les palmito-stéarates de PEG-9 à PEG-50 ; les béhénates de PEG-9 à PEG-50 (en noms CTFA : PEG-9 behenate à PEG-50 behenate) ; et leurs mélanges.

L'émulsion directe (A1) peut aussi comprendre des tensioactifs additionnels. On peut aussi à titre de tensioactifs additionnels utiliser les sels d'acides gras ayant 8 à 30 atomes de carbone, comme par exemple les sels d'acide palmitique, l'acide stéarique, l'acide béhénique ; les esters gras de glycérol, comme par exemple le glycéryl stéarate ; les dérivés oxyéthylénés des sels d'acides gras et des esters gras de glycérol, comportant 2 à 8 groupes oxyde d'éthylène, et leurs mélanges.

De préférence, les tensioactifs non-ioniques oxyéthylénés et/ou glycérolés de HLB allant de 8 à 18 sont choisis parmi les alcools gras en C18-C22 possédant de 6 à 12, et de préférence 8 à 12 moles d'oxyde d'éthylène.

Encore plus préférentiellement, le système émulsionnant de l'émulsion (A1) ne contient pas d'autres émulsionnants, que les alcools gras en C18-C22 possédant de 6 à 12 moles d'oxyde d'éthylène

De manière générale, le ou les tensioactifs non-ioniques oxyéthylénés et/ou glycérolés de HLB allant de 8 à 18 sont présents en une quantité allant de 0.2 à 30 %, de préférence de 0,5 à 20 % et mieux de 1 à 10 % en poids par rapport au poids total de la composition finale.

Le rapport système émulsionnant/phase lipophile va de préférence de 0,04 à 0,8, encore plus préférentiellement de 0,1 à 0,5. Comme indiqué plus haut, on entend par phase lipophile l'ensemble des constituants, qui ne sont pas hydrophiles et qui sont différents des tensioactifs non-ioniques oxyéthylénés et/ou glycérolés de HLB allant de 8 à 18.

La composition finale selon l'invention comprend de préférence une quantité de phase aqueuse allant de 30 à 95% du poids total de la composition finale, cette quantité pouvant aller encore plus préférentiellement de 50 à 95 % en poids.

Cette phase aqueuse se partage entre la phase aqueuse de l'émulsion directe (A1) et la phase aqueuse contenant le ou les polymères cationiques.

De manière classique, la phase aqueuse peut contenir, outre l'eau, un ou plusieurs solvants hydrosolubles choisis parmi les polyols (ou alcools polyhydriques), les alcool(s) inférieur(s) hydrosoluble(s) et leurs mélanges. On entend par alcool inférieur, un alcool comportant de 1 à 8 atomes de carbone. Comme polyols, on peut citer par exemple la glycérine ; les glycols comme le propylène glycol, le butylène glycol, l'isoprène glycol et les polyéthylène glycols tels que le PEG-8 ; le sorbitol ; les sucres tels que le glucose, le fructose, le maltose, le lactose, le sucrose ; et leurs mélanges. Comme alcools inférieurs, on peut citer par exemple l'éthanol, l'isopropanol, le butanol et leurs mélanges. Lorsqu'ils sont présents dans la composition de l'invention, le ou les solvant(s) peuvent être en une quantité allant de 0,01 à 60 % en poids, de préférence de 0,5 à 50 % en poids et mieux de 5 à 20 % en poids par rapport au poids total de la phase aqueuse. Ces adjuvants ainsi que leurs concentrations doivent être tels qu'ils ne modifient pas la propriété recherchée pour la composition de l'invention.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères utilisables, on peut notamment citer :
(1) Les homopolymères ou copolymères dérivés d'esters ou d'amides acryliques ou méthacryliques, réticulés ou non et comportant au moins un des motifs de formules (I), (II), (III) ou (IV) suivantes: 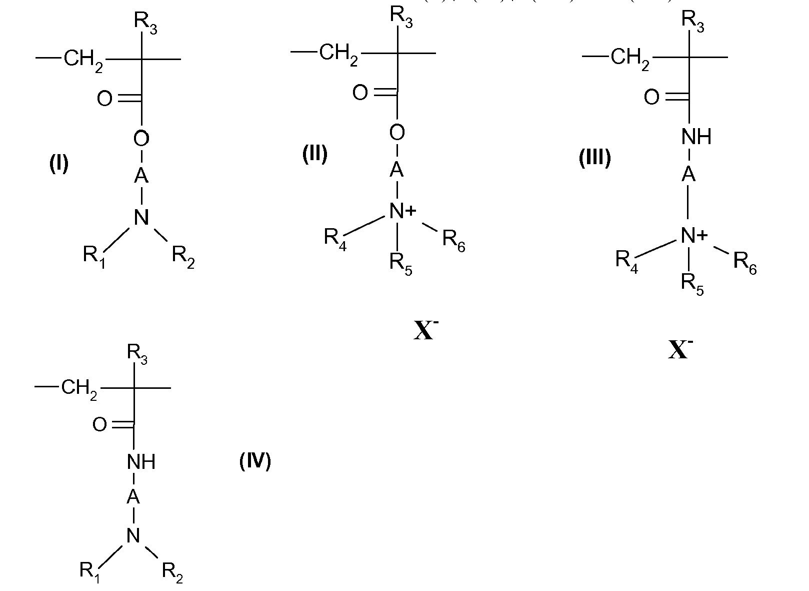 dans lesquelles:
   R₃ désigne un atome d'hydrogène ou un radical CH₃;
   A représente un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 6 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X⁻ désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un halogénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - les copolymères vinylpyrrolidone / méthacrylamide de diméthylaminopropyle quaternisés tel que le produit vendu sous la dénomination "GAFQUAT HS 100" par la société ISP,
   - les polymères réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société CIBA.
(2) Les polysaccharides cationiques notamment choisis parmi :
   a) les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
   b) les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
      Les produits commercialisés répondant à cette définition sont plus particulièrement les produits vendus sous la dénomination "Celquat L 200" et "Celquat H 100" par la Société National Starch.
   c) les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(3) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(4) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(5) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylamino hydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone. Le rapport molaire entre le polyalkylène polyamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique /époxypropyl/diéthylène-triamine.
(7) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 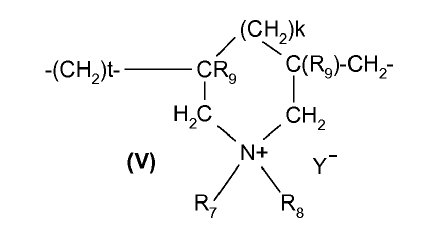 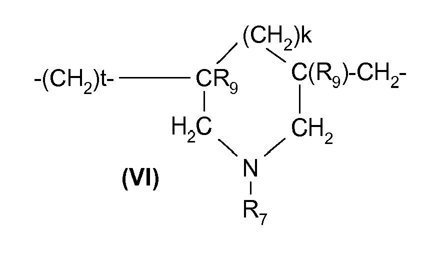 formules dans lesquelles
   k et t sont égaux à 0 ou 1, la somme k + t étant égale à 1 ;
   R₉ désigne un atome d'hydrogène ou un radical méthyle ;
   R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ;
   Y- est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallyl ammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(8) Le polymère de diammonium quaternaire contenant des motifs récurrents répondant à la formule (VII): 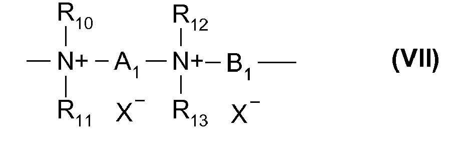 formule (VII) dans laquelle :
   R₁₀, R₁₁, R₁₂ et R₁₃, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃représentent un radical alkyle en C₁-C₆ linéaire ou ramifié substitué par un groupement nitrile, ester, acyle, amide ou -CO-O- R₁₄-D ou -CO-NH- R14-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement

      -(CH₂)ₙ-CO-D-OC-(CH₂)n-
   dans lequel n est compris entre 1 et 100 et de préférence entre 1 et 50, et D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)ₓ-CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂-CH₂-S-S-CH₂-CH₂- ;
   d) un groupement uréylène de formule : -NH-CO-NH- .
   De préférence, X- est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 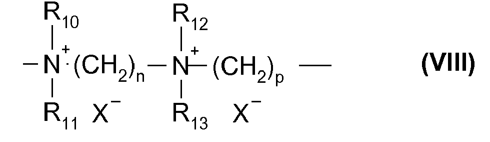 dans laquelle R₁₀, R₁₁, R₁₂ et R₁₃, identiques ou différents, désignent un radical alkyle ou hydroxyalkyle ayant de 1 à 4 atomes de carbone environ,
   n et p sont des nombres entiers variant de 2 à 20 environ et,
   X- est un anion dérivé d'un acide minéral ou organique.
(9) Les polymères de polyammonium quaternaire constitués de motifs de formule (IX) : 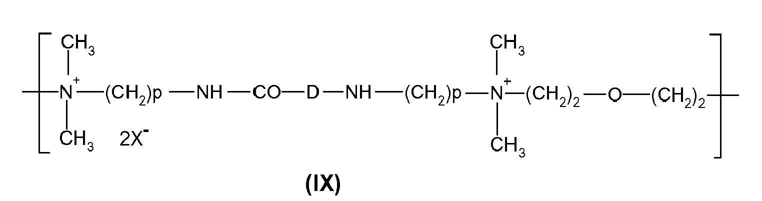 dans laquelle :
   p désigne un nombre entier variant de 1 à 6 environ,
   D peut être nul ou peut représenter un groupement -(CH₂)ᵣ -CO - dans lequel r désigne un nombre égal à 4 ou à 7, et
   X- est un anion dérivé d'un acide minéral ou organique.
   Les polymères cationiques comportant des motifs de formule (IX) sont notamment décrits dans la demande de brevet EP-A-122 324 et peuvent être préparés selon les procédés décrits dans les brevets U.S.A. n° 4 157 388, 4 390 689, 4 702 906, 4 719 282.
   Parmi ces polymères, on préfère ceux de masse moléculaire mesurée par RMN du Carbone 13 inférieure à 100000, et dans la formule de laquelle :
   p est égal à 3, et,
      a) D représente un groupement -(CH₂)₄ -CO- , X désigne un atome de chlore, la masse moléculaire mesurée par RMN du Carbone 13 ( RMN13C ) étant d'environ 5600 ; un polymère de ce type est proposé par la société MIRANOL sous le nom de MIRAPOL-AD1,
      b) D représente un groupement -(CH₂)₇ -CO - , X désigne un atome de chlore, la masse moléculaire mesurée par RMN du Carbone 13 ( RMN13C ) étant d'environ 8100 ; un polymère de ce type est proposé par la société MIRANOL sous le nom de MIRAPOL-AZ1,
      c) D désigne la valeur zéro, X désigne un atome de chlore, la masse moléculaire mesurée par RMN du Carbone 13, ( RMN13C ) étant d'environ 25500 ; un polymère de ce type est vendu par la société MIRANOL sous le nom MIRAPOL-A15,
      d) un " Block Copolymer " formé de motifs correspondant aux polymères décrits aux alinéas a) et c), proposé par la société MIRANOL sous les noms MIRAPOL-9, (masse moléculaire RMN13C, environ 7800) MIRAPOL-175, (masse moléculaire RMN13C, environ 8000) MIRAPOL-95, (masse moléculaire RMN13C, environ 12500).
   Plus particulièrement encore, on préfère selon l'invention le polymère à motifs de formule (XI) dans laquelle p est égal à 3, D désigne la valeur zéro, X désigne un atome de chlore, la masse moléculaire mesurée par RMN du Carbone 13, ( RMN13C ) étant d'environ 25500.
(10) Les polymères quaternaires de vinylpyrrolidone et de vinylimidazole tels que par exemple les produits commercialisés sous les dénominations Luviquat FC 905, FC 550 et FC 370 par la société B.A.S.F.
(11) Les polyamines comme le Polyquart H vendu par HENKEL, référencé sous le nom de " POLYETHYLENEGLYCOL (15) TALLOW POLYAMINE " dans le dictionnaire CTFA.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (7), (8).

Le ou les polymère(s) cationique(s) selon l'invention se trouve(nt) en une quantité de 0,01 à 10% en poids, de préférence de 0,05 à 5% en poids, et plus particulièrement de 0,1 à 2% en poids par rapport au poids total de la composition finale.

La composition selon la présente invention comporte un rapport pondéral de la quantité d'alcool gras sur la quantité de polymères cationiques supérieur à 1, de préférence compris entre 3 et 20, et encore plus préférentiellement compris entre 5 et 10.

### Additifs

La composition selon l'invention peut aussi contenir au moins un additif cosmétique couramment utilisé dans la technique tel que, par exemple, les silicones, les agents épaississants, les adoucissants, les agents anti-mousses, les agents hydratants, les agents émollients, les plastifiants, les pigments, les charges minérales, les argiles, les minéraux colloïdaux, les nacres, les parfums, les peptisants, les conservateurs, les polymères fixants ou non, les polymères conditionneurs autres que les polymères cationiques définis ci-dessus, les protéines et les vitamines.

Les quantités de ces différents adjuvants sont celles classiquement utilisées dans le domaine considéré, et par exemple de 0,01 à 20 % du poids total de la composition.

Bien entendu, l'homme de l'art veillera à choisir le ou les éventuels additifs de la composition selon l'invention de manière telle que les propriétés avantageuses attachées intrinsèquement à la composition conforme à l'invention ne soient pas ou substantiellement pas, altérées par l'addition envisagée.

La composition selon l'invention est de préférence une composition d'après-shampooing à rincer ou non, ou un shampooing.

Lorsque la composition se trouve sous la forme d'un shampooing, la composition comporte de préférence un ou plusieurs tensio-actifs anioniques et/ou amphotères.

Les tensioactifs anioniques pouvant être utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyllactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éthercarboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins
ou alcalino-terreux, d'ammonium, d'amine ou d'aminoalcool.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (A) et (B) :

Rₐ-CONHCH₂CH₂-N(R_{b})(R_{c})(CH₂COO⁻) (A)

dans laquelle:
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

   Rₐ'-CONHCH₂CH₂-N(B)(B') (B)
dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Elle peut aussi comprendre un ou plusieurs tensioactifs non-ioniques différents des tensioactifs non-ioniques de l'invention. En particulier, le tensio-actif peut être choisi parmi les alkylpolyglucosides.

Les compositions sous forme d'après-shampooing peuvent éventuellement comprendre un ou plusieurs agents tensioactifs cationiques, de préférence non polymérique, non polymérique signifiant sans la répétition dans sa structure d'au moins une unité issue de la polymérisation d'au moins un monomère.

A titre d'exemples de tensioactif cationique, on peut notamment citer les sels d'amines grasses primaires, secondaires ou tertiaires ; les sels d'ammonium quaternaire tels que les chlorures ou les bromures de tétraalkylammonium, d'alkylamidoalkyl trialkylammonium, de trialkylbenzylammonium, de trialkylhydroxy alkylammonium ou d'alkylpyridinium ; les dérivés d'imidazoline ; ou les oxydes d'amines à caractère cationique.

De préférence, les compositions selon l'invention ne contiennent pas de tensioactifs cationiques.

Les concentrations en tensioactifs autres que ceux de l'invention peuvent aller de 0,1 à 60% en poids par rapport au poids de la composition finale, mieux de 0,2 à 40%. De préférence, lorsque la composition est un shampooing, cette concentration varie entre 4 et 30%.

Le procédé de préparation des compositions de l'invention consiste à :
- préparer une émulsion directe (A1) comprenant un ou plusieurs alcools gras et un ou plusieurs tensioactifs non ioniques oxyéthylénés et/ou glycérolés de HLB allant de 8 à 18,
- ajouter cette émulsion à une composition aqueuse (A2) comprenant un ou plusieurs polymères cationiques.

La préparation de l'émulsion directe (A1) passe par une phase de chauffage au-dessus de la température d'inversion de phase du ou des tensioactifs de HLB allant de 8 à 18 et de la température de fusion du ou des alcools gras puis par une phase de refroidissement en dessous de la température d'inversion de phase. Le mélange avec la phase aqueuse (A2) peut se faire à une température comprise entre la température ambiante et la température d'inversion de phase. Il se fait de préférence à température ambiante.

De préférence, le rapport pondéral (A1)/(A1 + A2) va de 0,1 à 0,9 et de préférence de 0,15 à 0,5.

De manière plus détaillée, on peut opérer de manière suivante :
1) Peser dans un récipient tous les constituants de l'émulsion directe (A1)
2) Homogénéiser le mélange, par exemple au moyen d'un Rayneri 350 tours/min, et chauffer en augmentant progressivement la température au moyen d'un bain marie jusqu'à une température supérieure à la température d'inversion de phase T1, c'est-à-dire jusqu'à l'obtention d'une phase transparente ou translucide (zone de microémulsion ou de phase lamellaire) puis d'une phase plus visqueuse qui indique l'obtention de l'émulsion inverse (E/H).
3) Arrêter le chauffage et maintenir l'agitation jusqu'à retour à la température ambiante, en passant par la température d'inversion de phase T1, c'est-à-dire la température à laquelle se forme une émulsion H/E fine.
4) Lorsque la température est redescendue en dessous de la zone d'inversion de Phase en Température (T1), additionner le ou les polymères cationiques et les éventuels additifs et en particulier les éventuels tensio-actifs additionnels, et les matières premières thermosensibles.

On obtient une composition finale stable dont les gouttelettes de phase lipophile sont fines avec des tailles de 10 à 200 nm, de préférence de 30 à 150 nm.

Dans la zone de formation d'une microémulsion (mélange translucide), les interactions hydrophiles et hydrophobes sont équilibrées car la tendance du tensioactif est de former aussi bien des micelles directes que des micelles inverses. Par chauffage au-delà de cette zone, il y a formation d'une émulsion E/H, car le tensioactif favorise la formation d'une émulsion eau dans huile. Puis lors du refroidissement en dessous de la zone d'inversion de phase, l'émulsion devient une émulsion directe (H/E).

L'émulsification par inversion de phase est expliquée en détail dans l'ouvrage T.Fôrster, W von Rybinski, A.Wadle, Influence of microemulsion phases on the preparation of fine disperse emulsions, Advances in Colloid and interface sciences, 58, 119-149, 1995 cité ici pour référence.

L'invention a encore pour objet un procédé de traitement cosmétique des matières kératiniques, telles que les cheveux consistant à appliquer sur celles-ci une composition telle que définie précédemment, puis à effectuer éventuellement un rinçage à l'eau.

La présente invention a également pour objet l'utilisation cosmétique de la composition telle que définie ci-dessus, pour le traitement cosmétique des matières kératiniques.

La présente invention a notamment pour objet l'utilisation cosmétique de la composition telle que définie ci-dessus, en tant que après-shampooing pour le soin des matières kératiniques.

La présente invention a enfin pour objet l'utilisation cosmétique de la composition telle que définie ci-dessus, en tant que shampooing pour le lavage des matières kératiniques.

Les exemples indiqués ci-dessous permettront de mieux comprendre l'invention sans toutefois présenter un caractère limitatif. Les quantités sont indiquées en % en poids sauf mention contraire. Dans ce qui suit, MA signifie Matière Active.

### EXEMPLES

Mode opératoire pour réaliser les compositions de l'invention :

### 1) Préparation d'une émulsion d'huile par le procédé PIT

### Emulsification

- Peser la phase lipophile, les tensio-actifs émulsionnants, Homogénéiser
- Peser la phase aqueuse séparément, Homogénéiser
- Chauffer la phase lipophile à T = 70 °C dans un bécher sous agitation magnétique.
- Chauffer la phase aqueuse à T = 70 °C dans un bécher sous agitation magnétique.
- Verser la phase aqueuse dans la phase lipophile sous agitation magnétique. L'émulsion se forme et le milieu devient blanc.

### Chauffage

- Augmenter le chauffage jusqu'à Tmax = 90°C. On observe que le milieu devient translucide à T1< Tmax.

### Refroidissement

- Laisser refroidir sous agitation magnétique.

### 2) Préparation d'une formulation de shampooing

- A température ambiante, mélanger les tensio-actifs anioniques et/ou amphotères,
- Ajouter la prédispersion de phase lipophile obtenue par le procédé PIT,
- Ajouter le polymère cationique, l'eau résiduelle, et éventuellement les autres additifs hydrosolubles.

### 3) Préparation d'une formulation de soin

- A température ambiante, mélanger la prédispersion obtenue par le procédé PIT à de l'eau pour atteindre la concentration en phase lipophile voulue,
- Ajouter le polymère cationique, l'eau résiduelle, et éventuellement les autres additifs hydrosolubles.

### Exemple de compositions:

### Exemple 1 : Soin capillaire

| Composition PIT (A1) | |
|---|---|
| Alcool béhénique oxyéthyléné (10 OE) (EMULGIN BA 10, vendu par COGNIS) | 10.7 % MA |
| Alcool Cétylstéarylique (C16/C18 : 50/50) (LANETTE O OR, vendu par COGNIS ) | 30 % MA |
| Mélange Myristate/Palmitate/Stéarate de Myristyle/Cétyle/Stéaryle) (MIRACETI LASERSON) | 6.4 % MA |
| Eau | qsp100 % |

| Composition 1 selon l'invention | |
|---|---|
| Composition PIT (A1) | 23.3 % |
| Chlorure de poly di-méthyl di-allyl ammonium à 40 % dans l'eau (MERQUAT 100, vendu par NALCO) | 1 % MA |
| Acide citrique | Qsp pH 5 |
| Eau | qsp100 % |

| Composition 2 comparative | |
|---|---|
| Composition PIT (A1) | 23.3 % |
| Acide citrique | Qsp pH 5 |
| Eau | qsp100 % |

1 gramme de chaque composition est appliqué sur des mèches de 2,5 grammes de cheveux naturels. Une fois la composition appliquée, les mèches sont rincées et soumis à une première évaluation. On observe sur cheveux humides que le toucher de la mèche traitée avec la composition 1 de l'invention est plus lisse par rapport à la mèche traitée avec la composition 2 comparative.

### Exemple 2 : Soin capillaire

### Composition PIT

| | |
|---|---|
| ALCOOL BEHENIQUE OXYETHYLENE (10 OE) (EUMULGIN BA 10 - COGNIS) | 10.7%MA |
| ALCOOL CETYLSTEARYLIQUE (C16/C18 50/50) (LANETTE O OR- COGNIS) | 30%MA |
| MELANGE MYRISTATE/PALMITATE/STEARATE DE MYRISTYLE/CETYLE/STEARYLE (MIRACETI - LASERSON) | 6.4%MA |
| EAU | QSP 100% |

### Composition 3 selon l'invention

| | |
|---|---|
| COMPOSITION PIT | 10% |
| HOMOPOLYMERE DE CHLORURE DE METHACRYLATE D'ETHYL TRI-METHYL AMMONIUM RETICULE, EN DISPERSION DANS UN MEL. D'ESTERS A 50 % (SALCARE SC96 - CIBA) | 0,50%MA |
| ACIDE CITRIQUE | Qsp pH 5 |
| EAU | QSP 100% |

## Revendications

1. Composition cosmétique aqueuse pour le traitement des matières kératiniques, telles que les cheveux, comprenant une émulsion directe (A1) obtenue par un procédé PIT et un ou plusieurs polymères cationiques, **caractérisée par le fait que** l'émulsion directe (A1) comporte :
a) une phase lipophile comprenant un ou plusieurs alcools gras,
b) un système émulsionnant comprenant un ou plusieurs tensio-actifs non-ioniques oxyéthylénés et/ou glycérolés de HLB allant de 8 à 18, et
c) une phase aqueuse,
le rapport pondéral de la quantité d'alcools gras sur la quantité de polymères cationiques étant supérieur à 1.

2. Composition selon la revendication 1, **caractérisée par le fait que** l'alcool gras est choisi parmi les alcools oléique, laurique, palmitique, myristique, béhénylique, stéarylique, cétylique, linoléique, linolénique, arachidonique et leur mélange.

3. Composition selon la revendication précédente, **caractérisée par le fait que** l'alcool gras est l'alcool cétylstéarylique.

4. Composition selon l'une quelconque des revendications précédentes, **caractérisée par** le fait le ou les alcools gras sont présents en une quantité allant de 0.2 à 40 %, de préférence de 2 à 20 % et mieux de 3 à 11 % en poids par rapport au poids total de la composition finale.

5. Composition selon la revendication précédente, **caractérisée par** le fait le tensioactif non-ionique oxyéthylénés et/ou glycérolés de HLB allant de 8 à 18 est un alcools gras en C18 à C22 et possédant de 6 à 12, et de préférence de 8 à 12 moles d'oxyde d'éthylène.

6. Composition selon la revendication précédente, **caractérisée par le fait que** le tensioactif non-ionique oxyéthylénés et/ou glycérolés de HLB allant de 8 à 18 est le beheneth-10.

7. Composition selon l'une quelconque des revendications précédentes, **caractérisée par le fait que** les tensio-actifs non-ioniques oxyéthylénés et/ou glycérolés de HLB allant de 8 à 18 sont présents, de préférence, en une quantité allant de 0.2 à 30 %, de préférence de 0,5 à 20 % et mieux de 1 à 10 % en poids par rapport au poids total de la composition finale.

8. Composition selon l'une quelconque des revendications précédentes, **caractérisée par le fait que** le polymère cationique est choisi parmi :
(1) Les homopolymères ou copolymères dérivés d'esters ou d'amides acryliques ou méthacryliques et comportant au moins un des motifs de formules (I), (II), (III) ou (IV) suivantes: 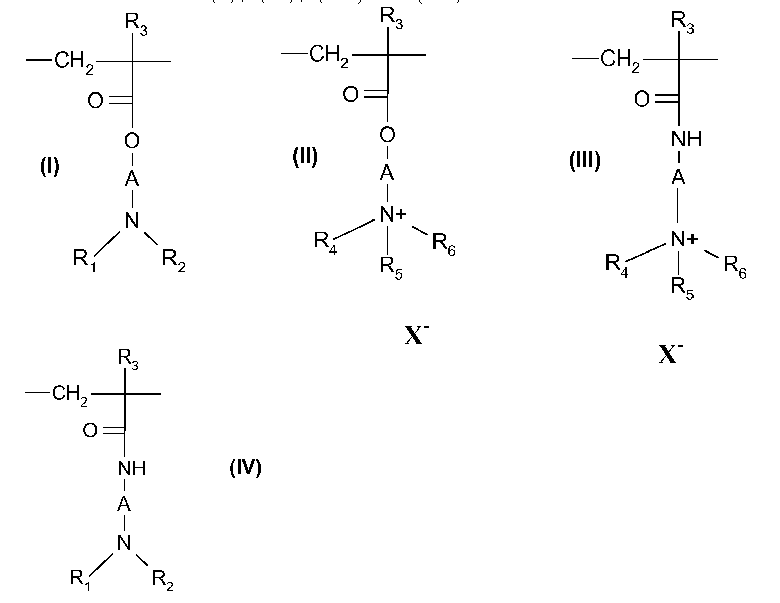 dans lesquelles:
R3 désigne un atome d'hydrogène ou un radical CH3;
A représente un groupe alkyle, linéaire ou ramifié, de 1 à 6 atomes de carbone, de préférence 2 ou 3 atomes de carbone ou un groupe hydroxyalkyle de 1 à 4 atomes de carbone ;
R4, R5, R6, identiques ou différents, représentent un groupe alkyle ayant de 1 à 6 atomes de carbone ou un radical benzyle et de préférence un groupe alkyle ayant de 1 à 6 atomes de carbone;
R1 et R2 , identiques ou différents, représentent hydrogène ou un groupe alkyle ayant de 1 à 6 atomes de carbone et de préférence méthyle ou éthyle;
X⁻ désigne un anion dérivé d'un acide minéral ou organique tel qu'un anion méthosulfate ou un halogénure tel que chlorure ou bromure.
(2) Les polysaccharides cationiques ;
(3) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères ;
(4) Les polyaminoamides solubles dans l'eau préparés en particulier par polycondensation d'un composé acide avec une polyamine ;
(5) Les dérivés de polyaminoamides résultant de la condensation de polyalcoylènes polyamines avec des acides polycarboxyliques suivie d'une alcoylation par des agents bifonctionnels.
(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone ;
(7) Les cyclopolymères d'alkyl diallyl amine ou de dialkyl diallyl ammonium ;
(8) Le polymère de diammonium quaternaire contenant des motifs récurrents répondant à la formule : 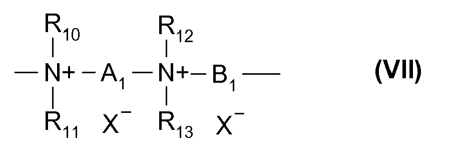 formule (VII) dans laquelle :
R10, R11, R12 et R13, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R10, R11, R12 et R13, ensemble ou séparément, constituent avec les atomes d'azote auxquels ils sont rattachés des hétérocycles contenant éventuellement un second hétéroatome autre que l'azote ou bien R10, R11, R12 et R13 représentent un radical alkyle en C1-C6 linéaire ou ramifié substitué par un groupement nitrile, ester, acyle, amide ou -CO-O- R14-D ou -CO-NH- R14-D où R14 est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R10 et R12 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement
-(CH2)n-CO-D-OC-(CH2)n-
dans lequel n est compris entre 1 et 100 et de préférence entre 1 et 50, et D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2- ;
d) un groupement uréylène de formule : -NH-CO-NH- .
(9) Les polymères de polyammonium quaternaire constitués de motifs de formule (IX) : 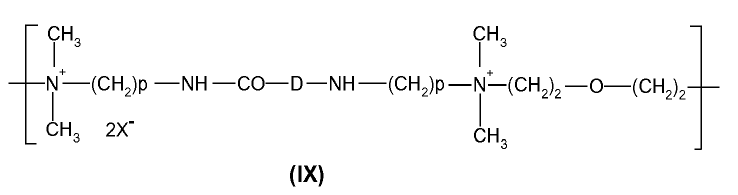 dans laquelle :
p désigne un nombre entier variant de 1 à 6 environ,
D peut être nul ou peut représenter un groupement -(CH2)r -CO - dans lequel r désigne un nombre égal à 4 ou à 7, et
X⁻ est un anion dérivé d'un acide minéral ou organique.
(10) Les polymères quaternaires de vinylpyrrolidone et de vinylimidazole
(11) Les polyamines.

9. Composition selon l'une quelconque des revendications précédentes, **caractérisée par le fait que** les polymères cationiques se trouvent en une quantité de 0,01 à 10% en poids par rapport au poids total de la composition finale.

10. Composition selon l'une quelconque des revendications précédentes, **caractérisée par le fait que** lorsque la composition se trouve sous la forme d'un shampooing, la composition comporte un ou plusieurs tensio-actifs anioniques et/ou amphotères.

11. Composition selon l'une quelconque des revendications précédentes, **caractérisée par le fait qu'**elle constitue une composition d'après-shampooing.

12. Procédé de préparation d'une composition telle que définie à l'une quelconque des revendications 1 à 11, consistant à :
- préparer une émulsion directe (A1) comprenant un ou plusieurs alcools gras et un ou plusieurs tensioactifs non ioniques oxyéthylénés et/ou glycérolés de HLB allant de 8 à 18,
le rapport pondéral de la quantité d'alcool gras sur la quantité de polymères cationiques étant supérieur à 1 ;
- ajouter cette émulsion à une composition aqueuse (A2) comprenant un ou plusieurs polymères cationiques.

13. Procédé de traitement des matières kératiniques, telles que les cheveux, **caractérisé par le fait que** l'on applique sur celles-ci une composition telle que définie à l'une quelconque des revendications 1 à 11, puis on effectue éventuellement un rinçage à l'eau.

14. Utilisation cosmétique d'une composition cosmétique telle que définie à l'une quelconque des revendications 1 à 11, en tant que après-shampooing pour le soin des matières kératiniques.

15. Utilisation cosmétique d'une composition cosmétique telle que définie à l'une quelconque des revendications 1 à 11, en tant que shampooing pour le lavage des matières kératiniques.
